(19) Europäisches Patentamt
European Patent Office
Office européen des brevets

(11) Veröffentlichungsnummer: **0 135 905**
A2

# (12) EUROPÄISCHE PATENTANMELDUNG

(21) Anmeldenummer: **84111096.8**

(22) Anmeldetag: **18.09.84**

(51) Int. Cl.⁴: **C 07 C 91/34,** A 61 K 31/135
// C07C93/14, C07C49/84,
C07C97/00, C07B57/00

(30) Priorität: **29.09.83 DE 3335217**
**31.03.84 DE 3412145**

(43) Veröffentlichungstag der Anmeldung: **03.04.85**
**Patentblatt 85/14**

(84) Benannte Vertragsstaaten: **AT BE CH DE FR GB IT LI LU NL SE**

(71) Anmelder: **Degussa Aktiengesellschaft,**
**Weissfrauenstrasse 9, D-6000 Frankfurt am Main 1 (DE)**

(72) Erfinder: **Klingler, Karl-Heinz, Dr., Beethovenstrasse 27,**
**D-6070 Langen (DE)**
Erfinder: **Engel, Jürgen, Dr., Cranachstrasse 25,**
**D-8755 Alzenau (DE)**
Erfinder: **Oepen, Gerhard, Dr.,**
**Fichtelgebirgsstrasse 129, D-6450 Hanau 6 (DE)**
Erfinder: **Stroman, Fritz, Dr.,**
**Herm.Steinhaüser-Strasse 18, D-6050 Offenbach (DE)**
Erfinder: **Thiemer, Klaus, Dr., Fürstenbergstrasse 12,**
**D-6450 Hanau 9 (DE)**
Erfinder: **Metzenauer, Peter, Dr.,**
**Fichtelgebirgsstrasse 131, D-6450 Hanau 6 (DE)**

## (54) Neue 1-(Alkyl-hydroxy-phenyl)-1-hydroxy-2-(alkylamino)-propane.

(57) Neue Verbindungen der Formel

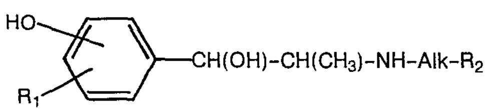

I

worin $R_1$ eine $C_1$–$C_4$-Alkylgruppe ist, $R_2$ einen gegebenenfalls durch Halogen, Hydroxygruppen, $C_1$–$C_6$-Alkylgruppen oder $C_1$–$C_6$-Alkoxygruppen substituierten Phenylrest oder einen gegebenenfalls durch $C_1$–$C_6$-Alkyl oder $C_1$–$C_6$-Alkoxy substituierten Cycloalkylrest mit 5 oder 6 Ringkohlenstoffatomen darstellt und Alk eine Alkylengruppe aus 3 oder 4 Kohlenstoffatomen bedeutet und deren Säureadditionssalze mit positiv inotroper Wirkung.

EP 0 135 905 A2

D e g u s s a Aktiengesellschaft
Weissfrauenstraße 9, 6000 Frankfurt/Main

Neue 1-(Alkyl-hydroxy-phenyl)-1-hydroxy-2-(alkylamino)-propane

Es ist bekannt, daß Hydroxyphenalaralkylaminoalkohole der allgemeinen Formel

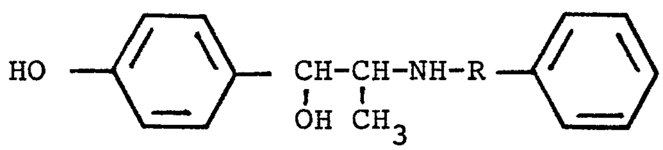

in der R ein Alkylenrest mit 2 bis 5 Kohlenstoffatomen ist, eine kräftige blutgefäßerweiternde Wirkung bei gleichzeitiger Erhöhung des Schlagvolumens und der Freiquenz des Herzens haben. Darüber hinaus besitzen diese Verbindungen eine uterospasmolytische Wirkung (siehe deutsche Patentschrift 11 82 245).

Die Erfindung betrifft die durch die Patentansprüche definierten Gegenstände.

Die erfindungsgemäßen Verbindungen sind pharmakologisch wirksam. Insbesondere besitzen sie günstige Kreislaufeffekte, insbesondere eine hohe positiv inotrope Wirkung. Bei einigen Verbindungen liegt nur eine geringfügige Beeinflussung der Herzfrequenz und des

...

Blutdrucks vor. Nach Versuchen am Ganztier stellen die erfindungsgemäßen Verbindungen fast reine $\beta^1$-Mimetica dar, während die aus der deutschen Patentschrift 11 82 245 bekannten Verbindungen (beispielsweise der bekannte Wirkstoff Buphenin) $\beta^2$-mimetisch wirken. Die erfindungsgemäßen Verbindungen unterscheiden sich in der Wirkung daher prinzipiell von den vorbekannten Verbindungen.

Bei den folgenden Angaben handelt es sich um erfindungswesentliche Erläuterungen.

Die in der Formel I vorkommenden Alkyl- und Alkoxygruppen können gerade oder verzweigt sein. $R_1$ ist insbesondere eine Methyl- oder Ethylgruppe, die vorzugsweise in 4-Stellung steht, falls die Hydroxygruppe sich in der 3-Stellung befindet. Bei den Alkylgruppen und Alkoxygruppen des Restes $R_2$ handelt es sich ebenfalls insbesondere um Alkyl- und Alkoxygruppen mit jeweils einem oder zwei Kohlenstoffatomen, insbesondere um Methyl- und Methoxygruppen. Vorzugsweise ist $R_2$ ein unsubstituierter Phenylrest. Als Halogenatome kommen beispielsweise in Frage: Chlor, Fluor und Brom, insbesondere Fluor und Chlor.
Falls $R_2$ ein Phenylrest ist, kann dieser einen, zwei oder drei der genannten Substituenten enthalten, wobei diese gleich oder verschieden sein können. Falls $R_2$ ein Cycloalkylrest ist, handelt es sich um den Cyclopentyl- oder Cyclohexylrest, die jeweils einen oder zwei der genannten Substituenten (gleiche oder verschiedene) enthalten können.
Die Gruppe Alk kann gerade oder verzweigt sein.

Beispiele für letztere sind: $-(CH_2)_3-$, $-(CH_2)_4-$, $-CH(CH_3)-CH_2-CH_2-$, $-CH_2-CH(CH_3)-CH_2-$, $-CH(CH_3)-CH_2-$.

...

Die Hydroxygruppe des linken Phenylrestes der Formel I befindet sich insbesondere in meta- oder para-Stellung. Der Rest $R_1$ befindet sich vorzugsweise in Nachbarstellung zu dieser Hydroxygruppe, wobei insbesondere die para-Stellung in Frage kommt, falls die Hydroxygruppe in meta-Stellung steht. Steht die Hydroxygruppe in para-Stellung, befindet sich $R_1$ vorzugsweise in meta-Stellung.
Besonders günstige Eigenschaften besitzt die Verbindung gemäß Beispiel 1.

Die erfindungsgemäßen Verbindungen enthalten in der Regel zwei oder auch drei asymmetrische Kohlenstoffatome. Bei der Synthese können daher Gemische von Diastereoisomeren auftreten. Man kann diese Gemische auf üblichem

...

Weg, beispielsweise durch Umkristallisieren, trennen. Die Enantiostereoisomeren lassen sich aus den reinen Racematen mit Hilfe optisch aktiver Hilfssubstanzen (zum Beispiel optisch aktive Säuren) in an sich bekannter Weise aufspalten. Es ist aber auch möglich, bei der Synthese optisch aktive beziehungsweise diastereomere Ausgangsstoffe einzusetzen, wobei die entsprechenden enantiostereomeren beziehungsweise die diastereomeren Endprodukte erhalten werden. Für die praktische pharmazeutische Verwendung kommen reine Isomere oder Isomerengemische in Frage.

Die Formel I umfasst sämtliche möglichen Enantiomere und Diastereomere.

Je nach den Verfahrensbedingungen und Ausgangsstoffen erhält man die Endstoffe der Formel I in freier Form oder in Form ihrer Salze. Die Salze der Endstoffe können in an sich bekannter Weise beispielsweise mit Alkali oder Ionenaustauschern wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen. Als solche Säuren seien beispielsweise genannt: Halogenwasserstoffsäuren, Schwefelsäure, organische Mono-, Di- oder Tricarbonsäuren der aliphatischen, alicyclischen, aromatischen oder heterocyclischen Reihe sowie Sulfonsäuren.

Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, oder Brenztraubensäure; Phenylessig-, Benzoe-, p-Amino-benzoe-, Anthranil-, p-Hydroxy-benzoe-, Salicyl- oder p-Aminosalicylsäure, Embonsäure, Methansulfon-, Äthansulfon-, Hydroxyäthansulfon-, Äthylensulfonsäure; Halogenbenzol-

...

sulfon-, Toluolsulfon-, Naphthalinsulfonsäure oder Sulfanilsäure oder auch 8-Chlor-theophyllin.

Bei den angegebenen Verfahren zur Herstellung der erfindungsgemäßen Verbindungen können die in Reaktion tretenden Aminogruppen bekannte und übliche Schutzgruppen enthalten. Es handelt sich hierbei um Reste, die durch Hydrolyse oder Hydrogenolyse leicht abspaltbar sind und häufig bereits während der Reaktion abgespalten werden. Falls solche Schutzgruppen bei der Verfahrensreaktion nicht abgespalten werden, dann erfolgt eine Abspaltung nach der Reaktion. Häufig enthalten die Ausgangsverbindungen aufgrund ihrer Herstellung bereits derartige Schutzgruppen.

Bei diesen Schutzgruppen handelt es sich beispielsweise um leicht solvolytisch abspaltbare Acylgruppen oder hydrierend abspaltbare Gruppen. Die solvolytisch abspaltbaren Schutzgruppen werden beispielsweise durch Verseifung mit verdünnten Säuren oder mittels basischer Substanzen (Pottasche, Soda, wässrige Alkalilösungen, alkoholische Alkalilösungen, $NH_3$) bei Temperaturen zwischen 10 und 150° C, insbesondere 20 - 100° C, abgespalten. Hydrierend abspaltbare Gruppen wie Arylalkylreste (Benzylrest) oder Hydroxycarbonylreste (Carbobenzoxyrest) werden zweckmäßig durch katalytische Hydrierung in Gegenwart üblicher Hydrierungskatalysatoren (Edelmetallkatalysatoren), insbesondere Palladium-Katalysatoren oder auch Platinkatalysatoren (Platinoxyd), Raney-Nickel, in einem Lösungs- oder Suspensionsmittel, gegebenenfalls unter erhöhtem Druck, (zum Beispiel 1 - 50 bar) bei Temperaturen zwischen 20 - 150° C, insbesondere 30 - 100° C, vorzugsweise 40 - 80° C abgespalten. Als Lösungs- beziehungsweise Suspensionsmittel kommen beispielsweise in Betracht: Wasser, niedere aliphatische Alkohole, cyclische Äther wie Dioxan oder Tetrahydrofuran, aliphatische Äther, Dimethylformamid und so weiter sowie Mischungen dieser Mittel.

...

Als Schutzgruppen, die durch Hydrogenolyse abspaltbar sind, kommen beispielsweise in Frage: Benzylrest, α-Phenyläthylrest, im Benzolkern substituierte Benzylreste (p-Brom- oder p-Nitrobenzylrest), Carbobenzoxyrest, Carbobenzthiorest, tert.-Butylhydroxycarbonylrest. Beispiele für hydrolytische abspaltbare Reste sind: Trifluoracetylrest, Phthalylrest, Tritylrest, p-Toluolsulfonylrest und ähnliche sowie niedere Alkanylreste wie Acetylrest, Formylrest, tert.-Butylcarboxyrest und ähnliche.

Insbesondere kommen die bei der Peptid-Synthese üblichen Schutzgruppen und die dort üblichen Abspaltungsverfahren in Frage. Unter anderem wird hierzu auch auf das Buch von Jesse P. Greenstein und Milton Winitz "Chemistry of Amino Acids", New York 1961, John Wiley and Sons, Inc. Volume 2, beispielsweise Seite 883 und folgende, verwiesen. Auch die Carbalkoxygruppe (zum Beispiel niedrigmolekulare) kommt in Frage.

Falls in den Ausgangsstoffen außerdem phenolische und/oder alkoholische Hydroxygruppen vorliegen, können diese ebenfalls durch die obengenannten Schutzgruppen geschützt sein, wobei die Abspaltung in der gleichen Weise erfolgt.

...

Zu dem Verfahren a)

Das Verfahren a) wird in einem Lösungs- oder Suspensionsmittel bei Temperaturen durchgeführt, die beispielsweise zwischen 20 bis 150° C, vorzugsweise 40 bis 150° C liegen. Beispielsweise arbeitet man in neutralem bis alkalischem pH-Bereich. Gegebenenfalls kann auch unter erhöhtem Druck (bis zu 150 bar) gearbeitet werden. Als Lösungs- oder Suspensionsmittel kommen beispielsweise in Betracht: Wasser, niedere aliphatische Alkohole mit 1 - 6 C-Atomen, gesättigte alicyclische und cyclische Äther mit 2 - 6 C-Atomen, niedere Alkylamide beziehungsweise Dialkylamide von aliphatischen $C_1$-$C_2$-Carbonsäuren, cyclische aliphatische Säureamide (5- oder 6-Ring) wie N-Methyl-pyrrolidon sowie Mischungen dieser Mittel.

Als Reduktionsmittel kommen in Betracht: Katalytisch aktivierter Wasserstoff unter Verwendung üblicher Metallkatalysatoren (mit und ohne Träger) wie Edelmetallkatalysatoren (Palladium, Palladiumkohle, Palladium auf Bariumsulfat, Platin, Platinoxid oder auch Raney-Nickel). Als Lösungsmittel kommen hierbei vorzugsweise polare Mittel wie Alkohole oder Alkohol-Wasser-Mischungen in Betracht. Die Reduktion ist aber auch möglich mit Leichtmetallhydriden, insbesondere komplexen Leichtmetallhydriden (Natriumhydrid, Lithiumhydrid, Natriumtriäthoxy-aluminiumhydrid, Natrium-bis-2-methoxy-ethoxy-aluminium-dihydrid, Lithium-tri-tert.-butoxy-aluminiumhydrid und ähnlichen), wobei als Lösungsmittel vorzugsweise alicyclische oder cyclische Ether verwendet werden bei Temperaturen von vorzugsweise 20 - 100° C.

...

Weiterhin kann die Reduktion mit Aluminiumamalgam oder Aluminiumalkoholaten (zum Beispiel Aluminiumisopropylat/ Isopropanol) und ähnlich wirkenden Mitteln oder durch elektrolytische Reduktion oder gegebenenfalls auch mit nascierendem Wasserstoff wie zum Beispiel Zink/Säure (Zink/Eisessig, Zink-Salzsäure) erfolgen.
Außer den bereits genannten Lösungsmitteln können auch noch aromatische Kohlenwasserstoffe (Benzol, Toluol) verwendet werden.

Vorhandene hydrogenolytisch abspaltbare Schutzgruppen (zum Beispiel Benzylgruppen) werden im allgemeinen bei der Reduktion abgespalten, falls diese Reduktion mittels Wasserstoff in Gegenwart von Hydrierungskatalysatoren, insbesondere Palladiumkatalysatoren, erfolgt. Falls vorhandene Schutzgruppen nicht während der Reaktion abgespalten werden, dann werden diese auf die bereits angegebene Weise nach Beendigung der Reaktion entfernt.

Die Herstellung der bei dem Verfahren a) verwendeten Ausgangsverbindungen der Formel II kann beispielsweise dadurch erfolgen, daß eine Verbindung der Formel

$$R_2 - Alk' - M \qquad VIII$$

mit einer Verbindung der Formel

...

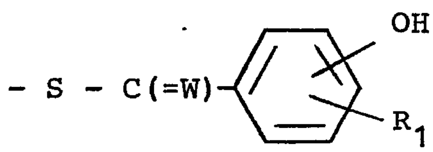

IX

kondensiert wird. In der Formel VIII bedeutet Alk' eine gerade oder verzweigte Alkylengruppe mit 2 oder 3 Kohlenstoffatomen, und M bedeutet entweder die Gruppe C(R)=O oder $-CHR-NH_2$, wobei R Wasserstoff oder eine Methylgruppe ist. In der Formel IX bedeutet S die Gruppe $-C(CH_3)=O$ oder die Gruppe $-CH(CH_3)NH_2.R_1$ und W hat die bereits angegebene Bedeutung. M und S sind dabei jeweils verschieden. Die primäre Aminogruppe in den Gruppen $-CHR-NH_2$ beziehungsweise $-CH(CH_3)-NH_2$ ist vorzugsweise durch eine der oben angegebenen Schutzgruppen, insbesondere eine hydrogenolytisch abspaltbare Schutzgruppe wie die Benzylgruppe geschützt. Die Kondensation der Verbindungen der Formeln VIII und IX wird im allgemeinen bei Temperaturen zwischen 20 und 150° C, insbesondere 40 bis 100° C, in einem Lösungs- oder Dispergiermittel wie niederen aliphatischen Alkoholen, Wasser-Alkohol-Gemischen, Dimethylformamid oder dimethylformamidhaltigen Lösungsmittelgemischen durchgeführt.

Es ist möglich, das durch Umsetzung der Verbindungen der Formel VIII mit Verbindungen der Formel IX erhaltene Reaktionsprodukt für die Umsetzung entsprechend dem Verfahren a) nicht erst zu isolieren, sondern sofort im gleichen Reaktionsmedium mit dem Reduktionsmittel

...

zu behandeln. Dies gilt insbesondere, wenn als Reduktionsmittel katalytisch aktivierter Wasserstoff verwendet wird. Dabei ist es ebenso gut möglich, daß das Reduktionsmittel, insbesondere katalytisch aktivierter Wasserstoff, bereits von Anfang an bei der Umsetzung der Verbindungen der Formel VIII mit Verbindungen der Formel IX vorhanden ist. Dementsprechend kann bei dem Verfahren a) als Ausgangsverbindung der Formel II auch ein Gemisch der Ausgangskomponenten der Formel VIII und IX verwendet werden.

Weiterhin können Ausgangsverbindungen der Formel II dadurch erhalten werden, daß eine Verbindung der Formel $R_2$-Alk-Hal oder $R_2$-Alk-O-Tosyl (p-Toluolsulfonyl-Rest), worin $R_2$ die angegebene Bedeutung hat, Alk eine gerade oder verzweigte Alkylengruppe mit 3 oder 4 C-Atomen ist, und Hal ein Halogenatom (vorzugsweise Chlor oder Brom) bedeutet, mit einer Verbindung

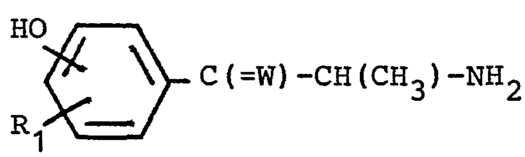

worin $R_1$ und W die angegebenen Bedeutungen haben und die primäre Aminogruppe auch durch eine übliche Schutzgruppe geschützt sein können, umsetzt. In analoger Weise kann auch eine Verbindung der Formel $R_2$-Alk-$NH_2$, worin $R_2$ und Alk die angegebenen genannten Bedeutungen haben und die Aminogruppe ebenfalls eine übliche Schutzgruppe

...

enthalten kann mit einer Verbindung der Formel

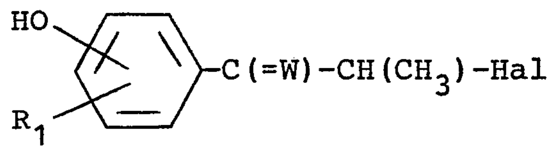

worin $R_1$ und W die angegebenen Bedeutungen haben und Hal ein Halogenatom (vorzugsweise Brom) ist, umgesetzt werden. Vorhandene Hydroxygruppen sind hierbei vorzugsweise ebenfalls durch übliche Schutzgruppen geschützt. Die letztgenannten Reaktionen werden beispielsweise mit oder ohne Lösungsmittel bei Temperaturen zwischen 20 und 200° C, vorzugsweise 50 - 180° C, durchgeführt, gegebenenfalls in Gegenwart von säurebindenden Mitteln. Als Lösungsmittel kommen beispielsweise in Betracht: Aromatische Kohlenwasserstoffe, aliphatische Ketone, Ether, Halogenkohlenwasserstoffe, Säureamide, Alkohole, Sulfoxyde.

...

Zu dem Verfahren b)

Das Verfahren b) kann ohne zusätzliches Lösungsmittel oder in einem geeigneten Lösungs- oder Dispergiermittel durchgeführt werden. Als Lösungs- oder Dispergiermittel kommen zum Beispiel in Betracht: Aromatische Kohlenwasserstoffe wie zum Beispiel Benzol, Mesitylen, Toluol, Xylol; niedere aliphatische Ketone wie zum Beispiel Aceton, Methyläthylketon; halogenierte Kohlenwasserstoffe wie zum Beispiel Chloroform, Tetrachlorkohlenstoff, Chlorbenzol, Methylenchlorid; Ether wie zum Beispiel Tetrahydrofuran und Dioxan; Sulfoxyde wie zum Beispiel Dimethylsulfoxid; tertiäre Säureamide wie zum Beispiel Dimethylformamid und N-Methylpyrrolidon; niedere Alkohole wie zum Beispiel Methanol, Äthanol, Isopropanol, Amylalkohol, Butanol, tert.-Butanol und so weiter. Die Reaktion wird beispielsweise bei Temperaturen von 20° bis 200° C, vorzugsweise 50 bis 180° C oder auch 80 bis 120° C, durchgeführt. Wird ein Lösungs- beziehungsweise Dispersionsmittel verwendet, arbeitet man häufig bei der Rückflußtemperatur dieses Mittels. Die Reaktion läuft häufig bereits auch schon bei Normaltemperatur ab, beziehungsweise bei einer Temperatur zwischen 20 bis 50° C.

Es kann zweckmäßig sein, eine der Ausgangsverbindungen im Überschuß einzusetzen und/oder die Reaktionskomponente der allgemeinen Formel III in gelöster beziehungsweise suspendierter Form zu der gelösten beziehungsweise suspendierten Reaktionskomponente der allgemeinen Formel IV zuzugeben. Das Molverhältnis

...

zwischen den Verbindungen der allgemeinen Formel III und IV kann 1 : 1 bis 1 : 10 und gegebenenfalls noch mehr betragen.

Bei der Durchführung der Reaktion kann als Ethylenoxid-Ausgangsverbindung anstelle der Äthylenoxid-Verbindung (falls in Formel III X in Form der Hydroxygruppe mit der benachbarten Hydroxygruppe den Ethylenoxidring bildet) auch das entsprechende Halogenhydrin oder ein Gemisch dieser beiden Verbindungen (Synthese-Rohprodukt) eingesetzt werden.

Gegebenenfalls kann die Umsetzung auch in Gegenwart von säurebindenden Mitteln wie Alkalicarbonaten, Pottasche, Soda, Alkalihydroxyden oder tertiären Basen durchgeführt werden.

Falls Z oder X eine veresterte Hydroxygruppe bedeutet, dann handelt es sich hierbei um reaktionsfähige Ester. Ein reaktionsfähiger Ester ist dabei zum Beispiel derjenige einer starken organischen oder anorganischen Säure, wie vor allem einer Halogenwasserstoffsäure, zum Beispiel der Chlor-, Brom- oder Jodwasserstoffsäure, oder einer Sulfonsäure, wie einer Aryl- oder $C_1$-$C_6$-Alkylsulfonsäure, zum Beispiel von niederen Alkylbenzolsulfonsäuren (p-Toluolsulfonsäure). Die Reaktion wird bei Verwendung eines reaktionsfähigen Esters vorteilhaft in Gegenwart eines basischen Kondensationsmittels oder eines Überschußes an Amin durchgeführt. Als Lösungsmittel kommen insbesondere Mittel wie Dioxan/Wasser, Dimethylformamid/Wasser oder niedere gesättigte aliphatische Alkohole in Frage.

Falls Z oder X eine Aminogruppe bedeutet, beziehungs-

...

weise falls Hydroxygruppen, insbesondere phenolische Hydroxygruppen, vorliegen, können diese eine der bereits angegebenen Schutzgruppen, beispielsweise eine Benzylgruppe, enthalten. Falls diese Schutzgruppen nicht bereits während der Reaktion abgespalten werden, können sie in der bereits angegebenen Weise nach Beendigung der Reaktion entfernt werden.

Die Ausgangsstoffe der Formel III sind bekannt oder werden aus den entsprechenden Keto-Verbindungen gemäß solchen Verfahren hergestellt, die beispielsweise in den folgenden Literaturstellen beschrieben sind: W.H. Hartung, J.C. Munch, E. Miller und F. Crossly, J.Am.Chem.Soc. 53, 1931, Seiten 4149-4160 (Ausgangsstoffe III mit X = $NH_2$); Houben-Weyl, Methoden der Organischen Chemie Band IV/1d (1981), Seiten 308-310 (Ausgangsstoffe III mit X = Halogen oder eine andere veresterte Hydroxygruppe. Letztere Verbindungen können außerdem analog Houben-Weyl, Methoden der Organischen Chemie, Band 5/3 (1962), Seite 503 ff., Band 6/2 (1963), Seite 475 ff. oder Band 9 (1955) Seite 426 hergestellt werden.

Die Ausgangsstoffe der Formel IV sind bekannt.

Verbindungen der Formel III, die einen Äthylenoxydring enthalten, sind bekannt beziehungsweise können in üblicher Weise aus Halogenhydriden der Formel III, worin X gleich Halogen ist, hergestellt werden (siehe hierzu Houben-Weyl, Methoden der Organischen Chemie, Band 6/3 (1965), Seite 374 und folgende): Behandlung des Halogenhydrins mit alkalischen Mitteln, beispielsweise alkoholischer KOH bei tieferen Temperaturen. Die phenolische Hydroxygruppe wird bevorzugt geschützt und die Schutzgruppen gegebenenfalls anschließend wieder entfernt.

...

Zu dem Verfahren c)

Die Abspaltung von Schutzgruppen gemäß diesem Verfahren erfolgt unter den hierfür bereits angegebenen Bedingungen.

Die Ausgangsstoffe der Formel V können beispielsweise gemäß den Verfahren a) und b) erhalten werden.

...

Pharmakologische beziehungsweise pharmazeutische Angaben

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.
Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor KG. Aulendorf in Württemberg 1971.

Beispiele hierfür sind Gelatine, natürliche Zucker wie Rohrzucker oder Milchzucker, Lecithin, Pektin, Stärke (zum Beispiel Maisstärke), Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel

...

Methyloxypropylcellulose, Methylcellulose, Hydroxypropylmethylcellulose), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Dimethylpolysiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen kommen beispielsweise Wasser oder physiologisch verträgliche organische Lösungsmittel

...

in Frage, wie zum Beispiel Ethanol, 1,2-Propylenglykol, Polyglykole und deren Derivate, Dimethylsulfoxyd, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: 1,3-Butandiol, Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Tragacanth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxyd pro Mol Glycerid).
Beispiele für Oleotriglyceride sind Olivenöl, Erdnussöl, Rhizinusöl, Sesamöl, Baumwollsaatöl, Maisöl (siehe auch Dr. H.P. Fiedler "Lexikon der Hilfsstoffe für Pharmazie, Kosmetik und angrenzende Gebiete" 1971, Seite 191 bis 195).

...

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.
Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

...

Die Applikation der Wirkstoffe beziehungsweise der Arzneimittel kann auf die Haut oder Schleimhaut oder in das Körperinnere erfolgen, beispielsweise oral, enteral, intravenös, intraarteriell, intrakardial, intramuskulär, intraperitoneal.
Bei den parenteralen Zubereitungsformen handelt es sich insbesondere um sterile beziehungsweise sterilisierte Erzeugnisse.

Der Zusatz anderer Arzneimittelwirkstoffe ist möglich.

Die erfindungsgemäßen Verbindungen zeigen beim narkotisierten Hund (Kreislaufversuch) eine gute positiv inotrope Wirkung sowie eine Herzzeitvolumenerhöhung. Beispielsweise wird bei obengenannter Versuchsmethode bei einer Dosis von 0,1 mg / Körpergewicht kg (Hund) eine 100 %ige Erhöhung der Herzmuskelkraft erzielt.

Die niedrigste, bereits inotrop wirksame Dosis in dem oben angegebenen Tierversuch ist beispielsweise

0,1 mg/kg oral
0,01 mg/kg intravenös.

Als allgemeiner Dosisbereich für die inotrope Wirkung (Tierversuch wie oben) kommt beispielsweise in Frage:

0,1 bis 1,0 mg/kg oral, insbesondere 0,3
0,01 bis 0,1 mg/kg intravenös, insbesondere 0,03.

Die erfindungsgemäßen Verbindungen sind daher zum Beispiel für die Behandlung von Myokard-Insuffizienz geeignet.

...

Die pharmazeutischen Zubereitungen enthalten im allgemeinen zwischen 0,01 mg bis 100 mg, vorzugsweise 0,1 bis 50 mg der erfindungsgemäßen aktiven Komponente(n).

Die Verabreichung kann beispielsweise in Form von Tabletten, Kapseln, Pillen, Dragees oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 5 mg und 50 mg oder Lösungen, die zwischen 0,1 % bis 5 % an aktiver Substanz enthalten.

Die Einzeldosis der erfindungsgemäßen aktiven Komponenten kann beispielsweise liegen

a) bei oralen Arzneiformen zwischen 5 mg und 50 mg vorzugsweise 10 bis 40 mg,

b) bei parenteralen Arzneiformen (zum Beispiel intravenös, intramuskulär) zwischen 0,5 mg und 5 mg vorzugsweise zwischen 2 mg und 4 mg.

- (Die Dosen sind jeweils bezogen auf die freie Base) -

Beispielsweise können 3 mal täglich 1 bis 3 Tabletten mit einem Gehalt von 10 bis 30 mg wirksamer Substanz oder zum Beispiel bei intravenöser Injektion 1 bis 5 mal täglich eine Ampulle von 1 bis 5 ml Inhalt mit 0,5 bis 5 mg Substanz empfohlen werden. Bei oraler Verabreichung ist die minimale tägliche Dosis beispielsweise 10 mg; die maximale tägliche Dosis bei oraler Verabreichung soll nicht über 100 mg liegen.

...

0135905

Für die Behandlung von Hunden und Katzen liegt die orale Einzeldosis im allgemeinen zwischen ungefähr 0,1 und 1,0 mg/kg Körpergewicht; die parenterale Dosis ungefähr zwischen 0,01 und 0,1 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 400 und 600 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

...

Beispiel 1

d,ℓ-1-(3-Hydroxy-4-methyl-phenyl)-1-hydroxy-2-(3-phenylpropylamino)-propan

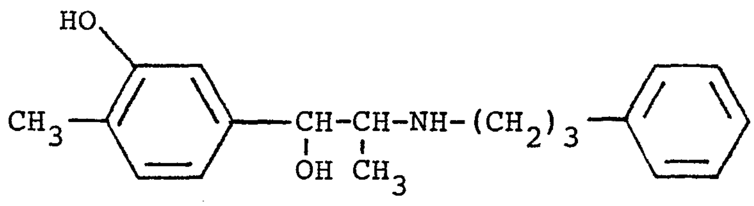

4,2 g 1-(3-Benzyloxy-4-methyl-phenyl)-1-oxo-2-(3-phenylpropylamino)-propan-hydrochlorid werden in 80 ml 90 %igem Ethanol unter Zusatz von 0,7 g 10 %igem Palladium-Kohle-Katalysator bei 50° C hydriert. Man filtriert, dampft ein und löst den Rückstand in Essigester. Das auskristallisierte Salz wird abgesaugt und getrocknet.
Ausbeute: 3,2 g
F. des Hydrochlorids: 160 - 161° C.

Herstellung der Ausgangsverbindung:

8,2 g 1-(3-Hydroxy-4-methyl-phenyl)-propanon-(1) werden zusammen mit 7,6 g Benzylchlorid, 8,4 g Kaliumcarbonat, 0,28 g Natriumjodid in 35 ml 95 %igem Ethanol bei Rückflußtemperatur umgesetzt. Nach sechsstündigem Sieden läßt man erkalten, versetzt mit Wasser, saugt ab und kristallisiert aus Isopropanol um.
Man erhält 12,5 g des entsprechenden 3-Benzyloxyketons (F.: 55 - 56° C).

...

11,5 g dieser Verbindung werden unter Stickstoff in 80 ml wasserfreiem Chloroform bei 50° C unter Rühren innerhalb von zwei Stunden mit einer Lösung aus 7,2 g Brom in 17 ml Chloroform versetzt. Man läßt im Stickstoffstrom abkühlen, dampft am Rotationsverdampfer ein und kristallisiert aus Isopropanol um.
Man erhält so 11,2 g 2-Brom-1-(3-benzyloxy-4-methylphenyl)-propanon-(1), welches bei 62 - 63° C schmilzt.

Ein Gemisch bestehend aus 10,0 g 2-Brom-1-(3-benzyloxy-4-methyl-phenyl)-propanon(1), 4,5 g 3-Phenylpropylamin, 40 ml Ethanol und 3,65 g Triethylamin wird 7 Stunden am Rückfluß gekocht und anschließend am Rotationsverdampfer eingedampft. Man nimmt den Rückstand in Wasser und Chloroform auf, trennt die Chloroformphase ab und schüttelt diese noch zweimal mit verdünnter Ammoniaklösung und zweimal mit Wasser aus. Der über Kaliumcarbonat getrocknete Extrakt wird eingedampft, der Rückstand in Essigsäureethylester gelöst und mit alkoholischer Salzsäure angesäuert. Das ausgefallene Hydrochlorid wird am nächsten Tag abgesaugt und aus Isopropanol umkristallisiert.
Das so erhaltene 1-(3-Benzyloxy-4-methyl-phenyl)-1-oxo-2-(3-phenyl-propylamino)-propan-hydrochlorid schmilzt bei 175 - 176° C.
Ausbeute: 5,4 g.

...

Analog Beispiel 1 werden die in Tabelle 1 aufgeführten d,ℓ-Verbindungen der Formel

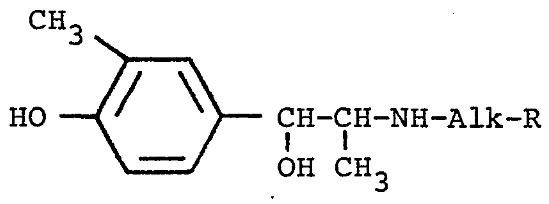

durch katalytische Hydrierung der entsprechenden geschützten d,ℓ-Ketone der Formel

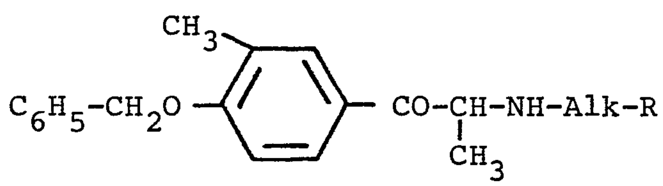

in Gegenwart eines 10 %igen Palladium-Kohle-Katalysators erhalten. Der nach Entfernen des Lösungsmittels erhaltene Rückstand wird jeweils aus Isopropanol umkristallisiert.

Die Herstellung der Ausgangsverbindung für die Beispiele 2 und 3 erfolgt analog Beispiel 1, wobei folgende Änderung vorgenommen wird: Das Kondensationsprodukt des 2-Brom-1-(3-methyl-4-benzyloxy-phenyl)-propanon-(1) mit 3-Phenyl-propylamin beziehungsweise 1-Methyl-3-phenyl-propylamin wird nicht in Chloroform sondern in einem Methylenchlorid-Wasser-Gemisch aufgenommen und der nach Abdampfen des Methylenchlorids erhaltene Rückstand wird in Aceton gelöst und mit Salzsäure angesäuert.

...

Tabelle 1

| Beispiel-Nr. | Alk | R | F. °C (Hydrochlorid) | Ausbeute g | Ausgangs-keton | Lösungsmittel | Katalysatormenge; Reaktionstemperatur |
|---|---|---|---|---|---|---|---|
| 2 | $-(CH_2)_3-$ | $-C_6H_5$ | 173–176 | 5,8 | 9 g | 150 ml 94 %iges Ethanol | 1 g; 45° C |
| 3 | $-CH(CH_3)(CH_2)_2-$ | $-C_6H_5$ | 221-222 | 4,5 | 9,1 g | 150 ml 94 %iges Ethanol | 1 g; 45° C |

Beispiel 4

d,ℓ-1-(3-Hydroxy-4-methyl-phenyl)-1-hydroxy-2-(3-p-fluor-phenyl-propylamino)-propan

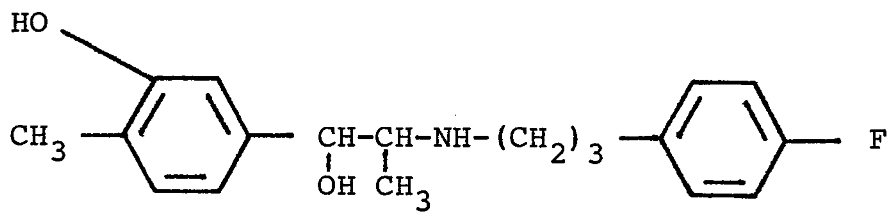

23,4 g 1-(3-Benzyloxy-4-methyl-phenyl)-1-oxo-2-(3-p-fluorphenyl-propyl-benzylamino)-propan-p-Toluolsulfonat (Rohprodukt, F. 140-142° C werden in Wasser und überschüssiger Natronlauge aufgenommen und zur Gewinnung der Base viermal mit Methylenchlorid ausgeschüttelt. Man destilliert das Lösungsmittel ab, löst den Rückstand in 130 ml Ethanol, säuert mit 1 n Salzsäure und hydriert bei 60° C unter Zusatz von 2 g 10 %igem Palladium-Kohle-Katalysator mit Wasserstoff unter Schütteln. Nach Beendigung der Wasserstoffaufnahme destilliert man ab und kristallisiert den Rückstand aus Aceton um. Man erhält 5,8 g des Hydrochlorids vom Schmelzpunkt 147-149° C.

Die Ausgangssubstanz wird wie folgt erhalten:
30 g p-Fluorphenyl-propyl-benzylamin-hydrochlorid werden mit 200 ml Wasser und überschüssiger konzentrierter Ammoniaklösung verrührt. Man schüttelt viermal mit Methylenchlorid aus und destilliert das Methylenchlorid ab. Der Rückstand wird in 240 ml Toluol gelöst, 17,9 g 2-Brom-1-(3-benzyloxy-4-methyl-phenyl)-propanon-(1) zugesetzt und die erhaltene Lösung

...

18 Stunden am Rückfluß gekocht. Man stellt über Nacht in den Kühlschrank und saugt das HBr-Salz des überschüssigen Ausgangsprodukts ab (14,9 g; F. 176-178° C). Das Toluolfiltrat wird im Vakuum abdestilliert, der Rückstand mit 10 %iger Ammoniaklösung alkalisch gestellt und viermal mit Methylenchlorid ausgeschüttelt. Die getrocknete Methylenchloridlösung wird eingedampft und der Rückstand in Aceton gelöst. Man säuert mit p-Toluolsulfonsäure an und fällt das entstandene p-Toluolsulfonat mit Petrolether aus (F. 140-142° C).

...

Beispiel 5

d, ℓ-1-(3-Methyl-4-hydroxy-phenyl)-1-hydroxy-2-/3-(3,4-dimethoxy-phenyl)-propylamino/-propan

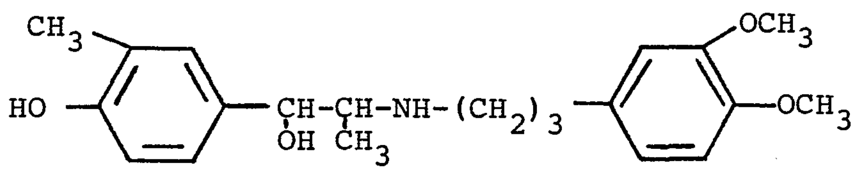

10,5 g 3-(3,4-Dimethoxyphenyl)-propanol-tosylat werden nun zusammen mit 7,7 g d, ℓ-3-Methyl-4-benzyloxy-norephedrin und 3,65 g Triethylamin in 60 ml n-Butanol umgesetzt. Man kocht 15 Stunden am Rückfluß, dampft im Rotationsverdampfer ein und nimmt den Rückstand in einem Chloroform-Wassergemisch auf. Man trennt die Phasen und schüttelt die Chloroform-Phase zweimal mit verdünnter Natronlauge und zweimal mit Wasser aus. Nach dem Trocknen mit Kaliumcarbonat wird filtriert, eingedampft, in Essigester gelöst und mit alkoholischer Salzsäure angesäuert. Am nächsten Tag wird abgesaugt und aus Ethanol umkristallisiert. Man erhält 6,6 g 1-(3-Methyl-4-benzyloxy-phenyl)-1-hydroxy-2-/3-(3,4-dimethoxyphenyl)-propylamino/-propan-hydro-chlorid, welches bei 198-200° C schmilzt.
Zur Abspaltung der Benzyl-Schutzgruppe werden 5,2 g der erhaltenen Benzylverbindung unter Zusatz von 0,5 g 10 %igem Palladium-Kohle-Katalysator in 55 ml 90 %igem Ethanol bei 50° C hydriert. Man filtriert, dampft ein und kristallisiert den Rückstand aus Isopropanol um.
F. des Hydrochlorids: 188-189° C
Ausbeute: 3,3 g.
Das d, ℓ-3-Methyl-4-benzyloxy-norephedrin kann wie folgt erhalten werden:

...

36,2 g d,ℓ-3-Methyl-4-hydroxy-norephedrin (vergleiche Beispiel 11) werden in einer aus 13,1 g 85 %igen KOH und 100 ml Ethanol hergestellten alkoholischen Kalilauge aufgenommen. Man dampft im Vakuum ein, versetzt mit 22,8 g Benzylchlorid in 220 ml Dimethylformamid und rührt 100 Stunden bei 20° C. Nun dampft man im Rotationsverdampfer im Vakuum ein, nimmt in 130 ml Wasser auf und säuert mit Salzsäure an (pH 3). Die filtrierte Lösung wird mit Natronlauge alkalisch gestellt und mit Chloroform ausgeschüttelt. Man trocknet die Chloroformphase mit Kaliumcarbonat, filtriert und dampft ein. Nach gründlichem Trocknen des Rückstandes im Vakuumtrockenschrank erhält man 35,2 g d,ℓ-3-Methyl-4-benzyloxy-norephedrin (F.: 95-96° C, Ausbeute: 65 % der Theorie).

...

Analog Beispiel 5 werden die in Tabelle 2 aufgeführten d,ℓ-Verbindungen der Formel

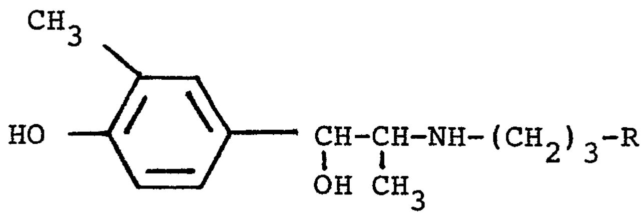

durch Umsetzung von d,ℓ-3-Methyl-4-benzyloxy-norephedrin und dem entsprechenden Tosylat des Propanols $R-(CH_2)_3-OH$ und anschließender Abspaltung der Benzylschutzgruppen durch Hydrierung in jeweils 55 ml 90 %igem Ethanol bei 50° C in Gegenwart von 0,5 g 10 %igem Palladium-Kohle-Katalysator erhalten. Bei den Beispielen 7 und 8 ist in dem Ausgangs-Tosylat die phenolische Hydroxygruppe ebenfalls durch eine Benzylgruppe geschützt. Bei Beispiel 9 werden für die Hydrierung von 15 Ausgangsverbindung 3 g Palladium-Katalysator verwendet (Lösungsmittel 15 ml Wasser und 300 ml Ethanol, Temperatur 58° C). Zur Herstellung der Ausgangssubstanz wird hier die Norephedrin-Verbindung mit dem 3-Cyclohexyl-propanol-tosylat 45 Stunden in 320 ml Butanol und 27 ml Triethylamin unter Rückfluß erhitzt.
Bei Beispiel 10 werden für die Hydrierung von 17,8 g Ausgangsverbindung 1,5 g Katalysator verwendet (120 ml 90 %iges Ethanol, Temperatur 55° C). Zur Herstellung der Ausgangssubstanz wird die Norephedrin-Verbindung mit dem entsprechenden Tosylat und 12,5 ml Triethylamin in 150 ml Ethanol 32 Stunden unter Rühren und Rückfluß erhitzt.

...

Tabelle 2

| Beispiel-Nr. | R | F. °C (Hydrochlorid) | Ausbeute g | Norephedrin Ausgangsverbindung | Ausgangs-Verbindung $R-(CH_2)_3-O-Tosylat$ | Ausbeute vor Abspaltung der Benzylgruppen |
|---|---|---|---|---|---|---|
| 6 | 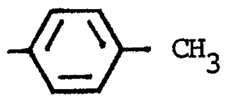 | 88-90 | 2,5 | 7,7 g | 9,1 g | 4,6 g |
| 7 | 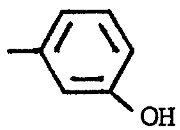 | 183-184 | 2,9 | 8,1 g | 12,3 g | 6,2 g |
| 8 | 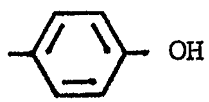 | 207-209 | 3,1 | 8,1 g | 12,3 g | 4,9 g |
| 9 | 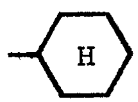 | 175-76 | 5,8 | 43,9 g | 48 g | 15,3 g |
| 10 | 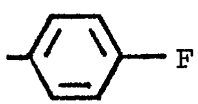 | 180-82 | 11,1 | 22 g | 25 g | 17,8 g |

Beispiel 11

ℓ-1-(3-Methyl-4-hydroxy-phenyl)-1-hydroxy-2-(3-phenylpropylamino)-propan

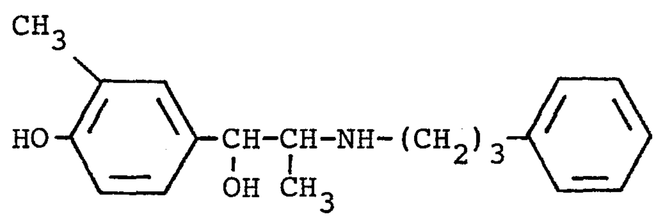

9,1 g ℓ-3-Methyl-4-hydroxy-norephedrin, 10,0 g 3-Phenylpropylbromid, 6,1 g Triethylamin und 50 ml Butanol werden 8 Stunden unter Rückfluß gekocht. Man destilliert das Lösungsmittel am Rotationsverdampfer ab und nimmt den Rückstand in einem Methylenchlorid-Wasser-Gemisch auf. Die abgetrennte Methylenchloridphase wird zweimal mit verdünnter Ammoniaklösung und einmal mit Wasser gewaschen, über Natriumsulfat getrocknet, filtriert und eingedampft. Die zurückbleibende Base löst man in Aceton und säuert mit alkoholischer Salzsäure an. Am nächsten Tag wird abgesaugt und getrocknet.
F. des Hydrochlorids: 181 - 182° C
Ausbeute: 5,2 g
Die linksdrehende Ausgangs-norephedrinverbindung wird wie folgt erhalten:

33,3 g 2-Brom-1-(3-methyl-4-benzyloxy-phenyl)-propanon-(1) werden zusammen mit 21,8 g Dibenzylamin, 10,4 g Triethylamin und 200 ml Toluol 12 Stunden unter Rühren am Rückfluß gekocht. Man versetzt mit 100 ml Wasser, trennt ab

...

und schüttelt die Toluolphase noch dreimal mit Wasser aus. Nach Trocknen mit Kaliumcarbonat wird eingedampft und der Rückstand aus 50 ml Ethanol umkristallisiert. Die so erhaltene Dibenzylverbindung (F.: 77-80° C; Ausbeute: 32,7 g) wird in einer alkoholischen Lösung (200 ml Ethanol und 8 ml Wasser) bei 55° C unter Zusatz von 2,0 g 10 %igem Palladium-Kohle-Katalysator hydriert. Man filtriert, dampft ein und trocknet im Vakuum. Die Ausbeute an d,$\ell$-3-Methyl-4-hydroxy-norephedrin ist 13,5 g.

200 g dieser d,$\ell$-Norephedrin-Base werden in 2 Liter Ethanol gelöst und unter Rühren mit 276,4 g (-)-N-(1-Methyl-2-phenyl-2-hydroxyethyl)-maleinsäuremonoamid versetzt. Man läßt über Nacht im Kühlschrank stehen und saugt das auskristallisierte Salz ab. Anschließend wird noch zweimal aus Ethanol umkristallisiert. Die so erhaltenen 123,6 g des reinen Salzes $[\alpha]_D^{20}$ + 10,5° werden mit 185 ml Wasser und 141,5 2n NaOH verrührt. Nach ca. 5 Stunden saugt man ab und kristallisiert aus Ethanol um. Man erhält 39 g $\ell$-3-Methyl-4-hydroxy-norephedrin, welches bei 171-173° C schmilzt. $[\alpha]_D^{20}$ (1g/100 ml Methanol): -15,9°

...

Beispiele für pharmazeutische Zubereitungen

Beispiel 1 / Tabletten

1 kg Wirkstoff der Verbindung gemäß Beispiel 7 wird mit 5 kg Milchzucker und 3 kg mikrokristalliner Cellulose gemischt und mit einer Lösung von 0,3 kg Polyvinylpyrrolidon in 1,2 kg Wasser in bekannter Weise granuliert.

Nach Zumischen von 3,45 kg mikrokristalliner Cellulose, 2 kg Maisstärke, 0,05 kg Hochdisperser Kieselsäure sowie 0,2 kg Magnesiumstearat werden Tabletten vom Gewicht 150 mg, einem Durchmesser von 7 mm und einem Wölbungsradius von 5 mm gepresst. Die Härte der Tabletten beträgt 4 - 7 kg (Heberlein-Härtetester). Jede Tablette enthält 10 mg Wirkstoff.

Beispiel 2 / Ampullen

15 g der Verbindung gemäß Beispiel 7 werden zusammen mit 87,7 g Natriumchlorid in 9 Litern Wasser für Injektionszwecke gelöst, die Lösung mit Wasser für Injektionszwecke auf 10 Liter aufgefüllt und über ein Membranfilter geeigneter Porenweite steril filtriert. Das Filtrat wird unter aseptischen Bedingungen zu 2 ml in sterilisierte Ampullen abgefüllt.

Eine Ampulle enthält 3 mg Wirksubstanz in 2 ml Lösung.

...

D e g u s s a Aktiengesellschaft
Weissfrauenstrasse 9, 6000 Frankfurt/Main

Neue 1-(Alkyl-hydroxy-phenyl)-1-hydroxy-2-(alkylamino)-propane

Patentansprüche:

1. Verbindungen der Formel

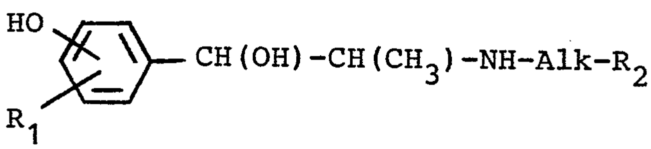 I

worin $R_1$ eine $C_1$-$C_4$-Alkylgruppe ist, $R_2$ einen gegebenenfalls durch Halogenatome, Hydroxygruppen, $C_1$-$C_6$-Alkylgruppen oder $C_1$-$C_6$-Alkoxygruppen substituierten Phenylrest oder einen gegebenenfalls durch $C_1$-$C_6$-Alkylgruppen oder $C_1$-$C_6$-Alkoxygruppen substituierten Cycloalkylrest mit 5 oder 6 Ringkohlenstoffatomen darstellt und Alk eine Alkylengruppe aus 3 oder 4 Kohlenstoffatomen bedeutet und deren Säureadditionssalze.

...

2. Verbindungen nach Anspruch 1, dadurch gekennzeichnet, daß $R_1$ in 4-Stellung und die phenolische Hydroxygruppe in 3-Stellung oder daß $R_1$ in 3-Stellung und die phenolische Hydroxygruppe in 4-Stellung steht.

3. Verbindungen nach einem oder mehreren der vorangegangenen Ansprüche, dadurch gekennzeichnet, daß $R_1$ eine Methylgruppe ist, Alk aus 3 C-Atomen besteht und $R_2$ Phenyl oder Halogenphenyl ist.

4. Verbindungen nach einem oder mehreren der vorangegangenen Ansprüche, dadurch gekennzeichnet, daß $R_2$ ein Phenyl-, ein Chlor-phenyl- oder ein Fluor-phenylrest ist.

5. Verbindungen nach einem oder mehreren der vorangegangenen Ansprüche, dadurch gekennzeichnet, daß $R_2$ ein Phenylrest oder ein 4-Chlor-phenyl oder ein 4-Fluor-phenylrest ist.

6. Verfahren zur Herstellung von Verbindungen der Formel

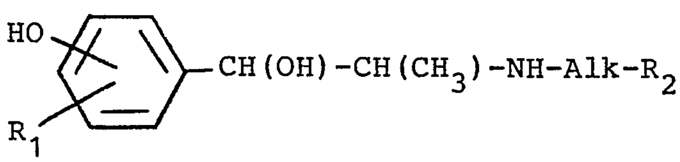 I

...

0135905

worin $R_1$ eine $C_1$-$C_4$-Alkylgruppe ist, $R_2$ einen gegebenenfalls durch Halogenatome, Hydroxygruppen, $C_1$-$C_6$-Alkylgruppen oder $C_1$-$C_6$-Alkoxygruppen substituierten Phenylrest oder einen gegebenenfalls durch $C_1$-$C_6$-Alkylgruppen oder $C_1$-$C_6$-Alkoxygruppen substituierten Cycloalkylrest mit 5 oder 6 Ringkohlenstoffatomen darstellt und Alk eine Alkylengruppe aus 3 oder 4 Kohlenstoffatomen bedeutet und deren Säureadditionssalzen,
dadurch gekennzeichnet,
daß man

a) eine Verbindung der allgemeinen Formel

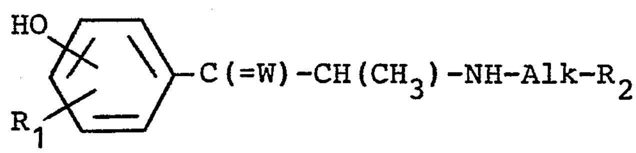

II

worin $R_1$, $R_2$ und Alk die angegebenen Bedeutungen haben, W Sauerstoff oder eine Hydroxygruppe plus ein Wasserstoffatom bedeutet, das sekundäre Stickstoffatom und/oder phenolische Hydroxygruppen auch geschützt sein können und wobei zwischen dem sekundären Stickstoffatom und der Gruppe Alk beziehungsweise der Gruppierung $-CH(CH_3)$ auch eine Doppelbindung vorliegen kann, die CO-Gruppe und/oder eine Doppelbindung reduziert und gegebenenfalls vorhandene Schutzgruppen abspaltet, oder

b) eine Verbindung der Formel

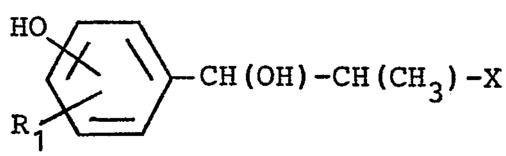

III

...

 0135905

mit einer Verbindung der Formel

$R_2$-Alk-Z IV

umsetzt, wobei in den Formeln III und IV R und Alk die angegebenen Bedeutungen haben, phenolische Hydroxygruppen auch geschützt sein können, und Z und X jeweils verschieden sind und entweder eine Aminogruppe beziehungsweise eine geschützte Aminogruppe oder eine durch eine starke anorganische oder organische Säure veresterte Hydroxygruppe bedeuten, wobei eine solche Hydroxygruppe in der Formel III in unveresterter Form zusammen mit der benachbarten Hydroxygruppe auch einen Ethylenoxidring bilden kann und gegebenenfalls in den erhaltenen Verbindungen vorhandene Schutzgruppen abspaltet, oder

c) in einer Verbindung der Formel

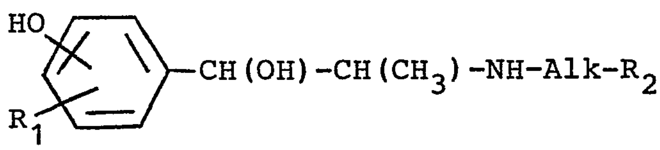 V

worin $R_1$, $R_2$ und Alk die angegebenen Bedeutungen haben und das sekundäre Stickstoffatom und/oder phenlische Hydroxygruppen geschützt sind, die vorhandene Schutzgruppen durch katalytische Hydrierung oder durch alkalische oder saure Verseifung abspaltet,

...

und gegebenenfalls die nach den Verfahren a) bis c) erhaltenen Verbindungen in die Säureadditionssalze überführt.

7. Verbindungen der Formel I zur Anwendung als therapeutische Wirkstoffe.

8. Arzneimittel, enthaltend eine Verbindung der allgemeinen Formel I neben üblichen Träger- und/oder Verdünnungs- beziehungsweise Hilfsstoffen.

9. Verfahren zur Herstellung eines Arzneimittels, dadurch gekennzeichnet, daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

10. Verwendung von Verbindungen der allgemeinen Formel I zur Herstellung von Arzneimitteln.

...